# EUROPEAN PATENT SPECIFICATION

(11) **EP 0 669 311 B1**
(45) Date of publication and mention of the grant of the patent: **22.04.1998**
(21) Application number: 95106899.8
(22) Date of filing: 28.01.1992
(51) Int. Cl.: C07C 65/26

(54) **Benzamides**
Benzamide
Benzamides

(30) Priority: 28.01.1991 GB 9101777; 16.08.1991 GB 9117727
(43) Date of publication of application: 30.08.1995
(62) Divisional of application: 92300726.4
(73) Proprietor: RHONE-POULENC RORER LIMITED, West Malling, Kent ME19 4TA (GB)
(72) Inventor: Ashton, Michael John, c/o Rhone-Poulenc Rorer Ltd., Dagenham, Essex RM10 7X (GB); Cook, David Charles, c/o Rhone-Poulenc Rorer Ltd., Dagenham, Essex RM10 7X (GB); Fenton, Garry, c/o Rhone-Poulenc Rorer Ltd., Dagenham, Essex RM10 7X (GB); Hills, Susan Jacqueline, c/o Rhone-Poulenc Rorer, Dagenham, Essex RM10 7X (GB); McFarlane, Ian Michael, c/o Rhone-Poulenc Rorer, Dagenham, Essex RM10 7X (GB); Palfreyman, Malcolm N., c/o Rhone-Poulenc Rorer, Dagenham, Essex RM10 7X (GB); Ratcliffe, Andrew James, c/o Rhone-Poulenc Rorer, Dagenham, Essex RM10 7X (GB); Vicker, Nigel, c/o Rhone-Poulenc Rorer Ltd., Dagenham, Essex RM10 7X (GB)
(74) Representative: Caffin, Lee

(56) References cited:
- PATENT ABSTRACTS OF JAPAN vol. 11, no. 398 (C-466) (2845) 25 December 1987 & JP-A-62 158 252 (KIRIN BREWERY CO. LTD.) 14 July 1987

## Description

This invention relates to a compound used in the preparation of therapeutically useful benzamide derivatives.

Thus, the present invention relates to 3-cyclopentyloxy-4-methoxybenzoic acid, a novel compound of general formula (Va) 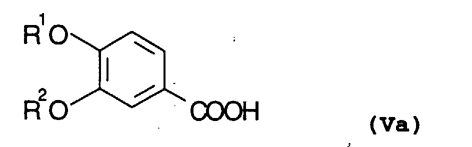 wherein R¹ represents methyl and R² represents cyclopentyl.

The compound of formula (Va) as defined hereinabove may be used in the preparation of compounds of general formula (I) 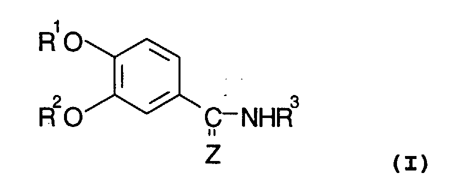 wherein R¹ and R² are as defined hereinabove, R³ represents an optionally substituted phenyl, naphthyl or heterocyclyl group, and Z represents an oxygen or sulphur atom; and when said heterocyclyl group contains one or more nitrogen ring atoms, N-oxides thereof, and pharmaceutically acceptable salts thereof.

The optional substituents within the R³ heterocyclyl group are one or more substituents selected from halogen atoms, alkyl groups which may carry one or more halogen atoms, aryl, arylalkyl, alkoxy, aryloxy, alkylthio, arylthio, alkoxycarbonyl, aryloxycarbonyl, alkanoyl, aroyl, alkylsulphonyl, arylsulphonyl, alkylsulphinyl, arylsulphinyl, hydroxy, hydroxyalkyl, formyl, alkanoylamino, aroylamino, cyano and nitro groups, and from amino, carbamoyl and sulphamoyl groups which themselves may each carry one or two alkyl substituents.

The optional substituents within the R³ phenyl and naphthyl groups are one or more substituents selected from halogen atoms and alkyl and alkoxy groups.

All alkyl groups and moieties, unless otherwise indicated, are straight- or branched chain and contain up to about 4 carbon atoms.

A preferred embodiment of the invention is the use of 3-cyclopentyloxy-4-methoxybenzoic acid in preparing the compound N-(3,5-dichloropyrid-4-yl)-3-cyclopentyloxy-4-methoxybenzamide and the N-oxide thereof, and pharmaceutically acceptable salts thereof.

The compounds of general formula (I) and their pharmaceutically acceptable salts exhibit pharmacological activity and accordingly are of use for the preparation of pharmaceutical compositions and in the treatment of humans and other animals. More especially, they are cyclic AMP phosphodiesterase inhibitors, in particular type IV cyclic AMP phosphodiesterase inhibitors. They are therefore useful, for example, as bronchodilators and asthma-prophylactic agents and agents for the inhibition of eosinophil accumulation and of the function of eosinophils, e.g. for the treatment of inflammatory airways disease, especially reversible airway obstruction or asthma, and for the treatment of other diseases and conditions characterised by, or having an aetiology involving, morbid eosinophil accumulation. As further examples of conditions which can be ameliorated by the administration of inhibitors of cyclic AMP phosphodiesterase such as compounds of general formula (I) there may be mentioned inflammatory diseases, such as atopic dermatitis, urticaria, allergic rhinitis, psoriasis, rheumatic arthritis, ulcerative colitis, Crohn's, disease, adult respiratory distress syndrome and diabetes insipidus, other proliferative skin diseases such keratosis and various types of dermatitis, conditions associated with cerebral metabo-lic inhibition, such as cerebral senility, multi-infarct dementia, senile dementia (Alzheimer's disease), and memory impairment associated with Parkinson's disease, and conditions ameliorated by neuroprotectant activity, such as cardiac arrest, stroke, and intermittent claudication.

Compounds of general formula (I) are prepared by the reaction of compounds of general formula (II) 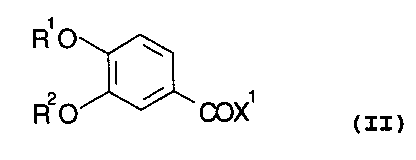 wherein R¹ and R² are as hereinbefore defined and X¹ represents a halogen, e.g. bromine or, preferably, chlorine atom, with compounds of the general formula (III)

NH₂R³ (III)

wherein R³ is as hereinbefore defined, preferably in the presence of a base, for example an alkali metal hydroxide or carbonate, e.g. sodium hydroxide or carbonate, an alkali metal hydride, e.g. sodium hydride, or an amine, preferably a tertiary amine, e.g. triethylamine or pyridine, optionally in an inert solvent, for example dichloromethane, dimethylformamide, or an ether, e.g. diethyl ether or tetrahydrofuran, preferably at a temperature from 0°C to the reflux temperature or at the melting point of the reaction mixture.

Compounds of formula (II) can be prepared from the compound of general formula (Va) as hereinbefore defined by the application or adaptation of known methods for the preparation of acid halides from carboxylic acids. For example, when X¹ represents a chlorine atom, the reaction can be carried out by means of thionyl chloride.

The compounds of formula (Va) can be prepared by the oxidation of the compound of general formula (VI) 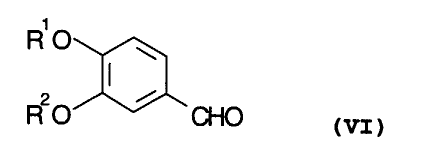 wherein R¹ and R² are as hereinbefore defined, e.g. by means of reaction with potassium permanganate, or with a mixture of sulphamic acid and sodium chlorite in acetic acid.

The compound of formula (VI) can be prepared from the compound of general formula (VII) 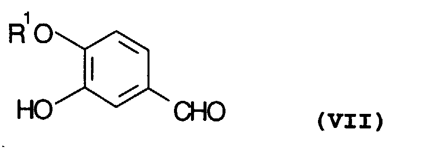 wherein R¹ is as hereinbefore defined by reaction with compounds of formula (V)

R²X² (V)

wherein R² is as hereinbefore defined and X² is halogen, or alternatively by reaction with compounds of the general formula (VIII)

R²OH (VIII)

wherein R² is as hereinbefore defined, preferably in the presence of a compound such as diisopropyl azodicarboxylate.

The following Examples illustrate the preparation of N-(3,5-dichloropyrid-4-yl)-3-cyclopentyloxy-4-methoxybenzamide via 3-cyclopentyloxy-4-methoxybenzoic acid.

In the nuclear magnetic resonance spectra (NMR) the chemical shifts are expressed in ppm relative to tetramethylsilane. Abbreviations have the following significances:s = singlet; d = doublet; t = triplet; q = quartet; m = multiplet; dd = doublet of doublets; b = broad.

### EXAMPLE 1

A stirred saturated aqueous solution of potassium permanganate (100ml) was treated with 3-cyclopentyloxy-4-methoxybenzaldehyde (7 .4g) and sodium carbonate (3.4g) and the mixture was stirred at 50°C for 1 hour, and then cooled to room temperature. The reaction mixture was acidified by treatment with concentrated hydrochloric acid and then it was treated with aqueous sodium bisulphite solution until a colourless solution was obtained. The reaction mixture was extracted with dichloromethane (2x100ml) and the organic extracts were dried over magnesium sulphate and concentrated. The resulting residue was recrystallised from diethyl ether, to give 3-cyclopentyloxy-4-methoxybenzoic acid (4.78g) in the form of white crystals. [NMR(CDCl₃) :- 1.7(s,2H),1.8-2.2 (m, 6H), 3.95(s,3H),4.85(s,1H), 6.9 (bs,1H),7.6(bs,1H), 7.8(s,1H),9.8(s,1H); Elemental analysis:- C,65.6; H,6.8%; Calculated:- C,66.1;H,6.8%].

### EXAMPLE 2

A solution of 3-cyclopentyloxy-4-methoxy benzaldehyde (66g) and sulphamic acid (39.6g) in glacial acetic acid (500ml) was treated dropwise during 1 hour with a solution of sodium chlorite (35g) in water (150ml). The mixture was stirred at 20°C during 1 hour and then it was treated with water (500ml) dropwise during 30 minutes. The resulting solid was filtered, washed with water and dried, to give 3-cyclopentyloxy-4-methoxybenzoic acid (60.9g) in the form of white crystals [Elemental analysis:- C,65.8; H,6.7%; calculated:- C,66.1;H,6.8%].

### EXAMPLE 3

Stirred thionyl chloride (20ml) was treated with 3-cyclopentyloxy-4-methoxybenzoic acid (5.0g) portionwise and the solution was then heated at 85°C for 3 hours. Toluene (50ml) was added and the mixture was concentrated to give 3-cyclopentyloxy-4-methoxybenzoyl chloride (4.12g) in the form of an oil which slowly crystallised. [NMR(CDCl₃): 1.6-1.7 (m,2H),1.8-1.95(m,4H),1.94-2.05(m,2H),3.94(s,3H),4.85 (m,1H),6.9(d,1H),7.55(d,1H),7.8(q,1H); Elemental analysis:- C,61.3;H,5.94;C1,13.9%; Calculated:- C,61.3;H,5.94;Cl,13.92%].

### EXAMPLE 4

A suspension of sodium hydride (60% dispersion in oil; 2.2g) in dry tetrahydrofuran (25ml) at 15-20°C was treated portionwise with a solution of 4-amino-3,5-dichloropyridine (4.5g) in dry tetrahydrofuran (40ml), with cooling. The mixture was stirred for a further 30 minutes, and then it was cooled to 10°C and treated with a solution of 3-cyclopentyloxy-4-methoxybenzoyl chloride (6.4g) in dry tetrahydrofuran (40ml), dropwise, during 45 minutes at 10°C. The mixture was stirred at 10°C for 30 minutes and was then treated with dilute hydrochloric acid (50ml;1N), followed by dichloromethane (75ml). The layers were separated and the aqueous layer was washed with a further quantity of dichloromethane (25ml). The combined organic layers were washed with water (50ml), with saturated aqueous sodium bicarbonate solution (100ml), and with water (50ml), dried over magnesium sulphate and evaporated to dryness. The resulting residue was recrystallisecl from isopropanol, to give N-(3,5-dichloropyrid-4-yl)-3-cyclopentyloxy-4-methoxybenzamide (7.0g).

### REFERENCE EXAMPLE 1

A stirred solution of 3-hydroxy-4-methoxybenzaldehyde (2.00g) in dry dimethylformamide (20ml) was treated portionwise with sodium hydride (60% in oil; 0.56g) and the mixture was then heated for 1 hour at 50°C. It was then treated dropwise with cyclopentyl bromide (2.36g) and was stirred and heated at 50°C for 22 hours. The solution was diluted with water (100ml) and extracted with diethyl ether (2x100ml). The ethereal extracts were combined, dried over magnesium sulphate and concentrated, to give 3-cyclopentyloxy-4-methoxybenzaldehyde (1.65g) in the form of a golden oil.

### REFERENCE EXAMPLE 2

A stirred solution of 4-aminopyridine (40g) in concentrated hydrochloric acid (500ml) at 80°C. was treated dropwise with aqueous hydrdgen peroxide solution (200ml;15%w/w), keeping the temperature between 80°C. and 85°C. The solution was then cooled and basified by dropwise treatment with aqueous sodium hydroxide solution (50∼w/w), keeping the temperature below 15°C. The resulting white flocculent precipitate was recrystallised from toluene, to give 4-amino-3,5-dichloropyridine -(61.5g), m.p. 161.5-162.5°C.

### REFERENCE EXAMPLE 3

A solution of 4-aminopyridine (47g) in concentrated hydrochloric acid (355ml) was treated portionwise at 80°C with an aqueous solution of sodium hypochlorite (550ml;15%w/v). The mixture was cooled to 30°C and basified by treatment with aqueous sodium hydroxide solution (300ml;35%w/v) during 20 minutes. The mixture was stirred and cooled for a further 30 minutes and then it was filtered. The solid was washed well with water and dried at 60°C, to give 4-amino-3,5-dichloropyridine (69.5g).

## Claims

1. 3-Cyclopentyloxy-4-methoxybenzoic acid.

## Patentansprüche

1. 3-Cyclopentyloxy-4-methoxybenzoesäure.

## Revendications

1. Acide 3-cyclopentyloxy-4-méthoxybenzoïque.
